# EUROPEAN PATENT APPLICATION

(11) **EP 2 532 735 A1**
(43) Date of publication of application: **12.12.2012**
(21) Application number: 11169159.8
(22) Date of filing: 08.06.2011
(51) Int. Cl.: C12N 5/00

(54) **Identifying optimal topography to control cellular function and neo-tissue formation**

(71) Applicant: National University of Ireland, Galway, Galway (IE)
(72) Inventor: Zeugolis, Dimitrios, Galway (IE); English, Andrew, Knocknacarra Galway (IE); Azeem, Ayesha, Enniscorthy County Wexford (IE)
(74) Representative: Gates, Marie Christina Esther

(57) **Abstract**

The invention is in the field of tissue engineering and regenerative medicine and in particular relates to the production of tissue scaffolds for replacing or repairing whole tissues. The invention identifyies the optimal topography to control cellular function and neo-tissue formation.

## Description

### Field of the Invention

The invention is in the field of tissue engineering and regenerative medicine and in particular relates to the production of tissue scaffolds for replacing or repairing whole tissues.

### Background to the Invention

Over 33 million musculoskeletal injuries per year in United States alone; almost 50% of them are tendon and ligament related with approximately 95,000 new cases per year. Musculoskeletal injuries and degenerative conditions constitute a bottleneck to the healthcare system with an associated expenditure ranging from €70 to €150 billion per year. Typically, around 50% of the European population reports musculoskeletal pain at one or more sites for at least one week every month.. Surgical repairs do not fully restore function due to fibrous adhesions or failure arising from the mechanical demands placed on imperfect integrative healing at tendon-tendon or tendon-bone interfaces [9, 10]. As the human population ages and the life expectancy increases, musculoskeletal injuries will continue to rise putting a further physical and financial strain on healthcare system. Therefore, to develop strategies for functional tissue regeneration is of paramount importance. Tissue engineering aims to mimic the native tissue properties with the ultimate goal being the fabrication of implants that would closely imitate native extracellular matrix assemblies and restore function.

Tissue grafts (autografts, allografts and xenografts) are currently the most commonly used substitutes to reconstruct injured tissues since they closely imitate the structural, biochemical, biophysical and biological properties of the tissue to be replaced. However, allografts/xenografts still face the risk of potential transmission of infectious diseases, poor success rate and possibilities of immune rejection, whilst autografts present the disadvantages of creating a secondary morbid site during harvesting and limited availability.

For tissue transplants, cells are often implanted or seeded into an artificial structure capable of supporting three dimensional tissue formation, which can then be implanted into the body. Scaffolds may also be implanted into the body at sites where repair is required, and body cells migrate to become deposited and grow on the scaffold, ultimately the growth repairing the tissue damage. These scaffold structures are critical to recreating *the in vivo* milieu and allowing cells to influence their own microenvironments. Scaffolds generally allow cell attachment and migration, they deliver and retain cells and biochemical factors, they enable diffusion of vital cell nutrients and expressed products and they exert mechanical and biological influences to modify the behaviour of the cells. In a scaffold, a high porosity and an adequate pore size are necessary to facilitate cell seeding and diffusion of both cells and nutrients throughout the whole structure. They should also be biodegradable to avoid the necessity of surgical removal. This means that as the cells fabricate their own natural matrix structure around themselves, the scaffold is able to provide structural integrity, but that it will eventually break down leaving the newly formed tissue, which will take over the mechanical load.

A number of different methods have been described in the literature for preparing porous structures to be employed as tissue engineering scaffolds. One of these is solvent casting in which a polymer is dissolved in a suitable organic solvent and the solution is cast into a mould filled with porogen particles. The size of the porogen particles will affect the size of the scaffold pores while the polymer to porogen ratio correlates with the amount of porosity of the final structure. After casting, the solvent is allowed to evaporate and the porogen is dissolved resulting in a porous structure.

Electro spinning is a versatile technique that can be used to produce continuous fibres from submicron to nanometre diameters. Generally a solution is fed through a spinerette and a high voltage is applied to the tip. The build up of electrostatic repulsion within the charged solution causes it to eject a thin fibrous stream. A mounted collector plate or rod with an opposite or grounded charge draws in the continuous fibres which arrive to form a highly porous network. This is a technique which is easy to vary so that modifying the distance to the collector, the magnitude of the applied voltage or the solution flow rate can dramatically change the overall scaffold architecture.

To this end, tissue engineering was pioneered as the only viable alternative to address the transplantation crisis. Several natural (e.g. collagen) and synthetic (e.g. PLGA, PLLA) polymers have been clinically translated over the years. Biomaterials design has also evolved from primitive materials that matched mechanical properties and durability to biofunctional materials that aim to incorporate instructive signals into scaffolds and modulate cellular functions, such as attachment and directional migration. Many biological processes such as cell attachment and migration, angiogenesis and neotissue formation and development are regulated by spatially-dependent signals. Therefore, incorporation of biophysical cues through scaffold architecture is expected to imitate the native extracellular matrix environment and ultimately enhance functional neotissue formation.

Indeed, several micro- [28-30] - and nano-[31-33] fabrication technologies of polymers in the field of tissue engineering have been demonstrated to achieve topographical, spatial, chemical and immunological control over cells and therefore create more functional tissue engineering constructs [24, 34-37]. Recent data demonstrate the superiority of nano-textured scaffolds over micro-textured scaffolds. Indeed, nano-textured scaffolds: **(a)** operate on the same small scale as all the functions involved in the growth, development and ageing of the human body; and **(b)** offer distinct advantages over micro-scaffolds such as directional cell growth that promotes functional neotissue formation; larger surface areas to adsorb proteins; and more binding sites to cell receptors [38-42]. Despite that, such technologies have still not been clinically translated, since we still cannot fabricate constructs with well-defined 2D and 3D surface topography. For example, although electro-spinning has been recently introduced as a method to produce aligned scaffolds that promote cellular attachment and migration, the voids between the fibres cannot be accurately controlled. To this end, patterning technologies [43-46] have been introduced to produce nano-textured constructs with well-defined topography at the nano-level. However, the ideal surface topography for specific cell type and for optimal cellular behaviour has not been identified. Thus, herein we show that different cells differentially respond to nano-topography and that there is an ideal nano-topography for optimal cellular behaviour and functional neotissue formation.

### Object of the Invention

It is thus an object of the invention to provide a scaffold having an optimized nano-topography for the formation of functional neotissue formation. A further object is to provide a scaffold which allows the alignment of cells in a manner closely resembling that of native body tissue formation. Among the major challenges now facing tissue engineering is the need for more complex functionality, as well as both functional and biomechanical stability in tissues which are stimulated for repair. Accordingly, a further object is to provide neotissue scaffold which stimulates or supports tissue formation with improved functional and biochemical stability. Tissue formed using the scaffold is suitable for tendon regeneration, bone regeneration, nerve generation or cornea regeneration.

### Summary of the Invention

According to the present invention there is provided a scaffold for the growth of tissue comprising a film of supporting material, wherein the film has a plurality of nano-grooves on its surface. The scaffold may have a groove width of about 2,200nm. The scaffold may have grooves which have a line width of about 1,800nm. The scaffold may have grooves which have a groove depth is about 300nm.

The supporting material is (poly)glycolide-co-lactide or a PLA/PCL.

A scaffold suitable for the growth of human osteosarcoma cells may have grooves which have a width of 2,200nm, a line width of 1,800nm and a groove depth of 300nm. A scaffold suitable for the growth of human fibroblasts may have grooves which have a width of 2,200nm, a line width of 1,800nm and a groove depth of 300nm.

A scaffold suitable for the growth of tenocytes may have grooves which have a width of 2,200nm, a line width of 1,800nm and a groove depth of 300nm.

The invention also provides use of a tissue scaffold having nano-grooves on the surface thereof in a method of repairing or replacing tissue in a patient.

The invention also relates to a method of repairing or replacing tissue in a patient comprising use of a tissue scaffold having nano-grooves on the surface thereof.

### Brief Description of the Drawings

**Figure 1****- Scaffold Architecture:** Top, from left to right: smooth films; porous films; porous non-aligned electro-spun scaffolds. Bottom, from left to right: porous aligned; non-aligned; and aligned electro-spun scaffolds.
**Figure 2** **-Biological response using human osteosarcoma cells:** Top, from left to right: smooth films; porous films; porous non-aligned electro-spun scaffolds. Bottom, from left to right: porous aligned; non-aligned; and aligned electro-spun scaffolds. Cells attached only on the non-aligned and aligned electro-spun scaffolds. Moreover, only aligned electro-spun mats promote cell alignment in the direction of the substrate topography.
**Figure 3** **- Biological evaluation using human osteosarcoma cells:** Cells exposed to nano-textured scaffolds exhibit significantly reduced metabolic activity after 10 and 14 days in culture.
**Figure 4A****-D:** AFM micrographs of scaffold option No 2 (2200x1800x35).
**Figure 5****:** Biological response of human osteosarcoma cells on non-grooved scaffolds. No alignment is observed
**Figure 6****:** Biological response of human osteosarcoma cells on scaffold option 1 (2200x 1 800x3 00). The cells align in the direction of the underlying topography.

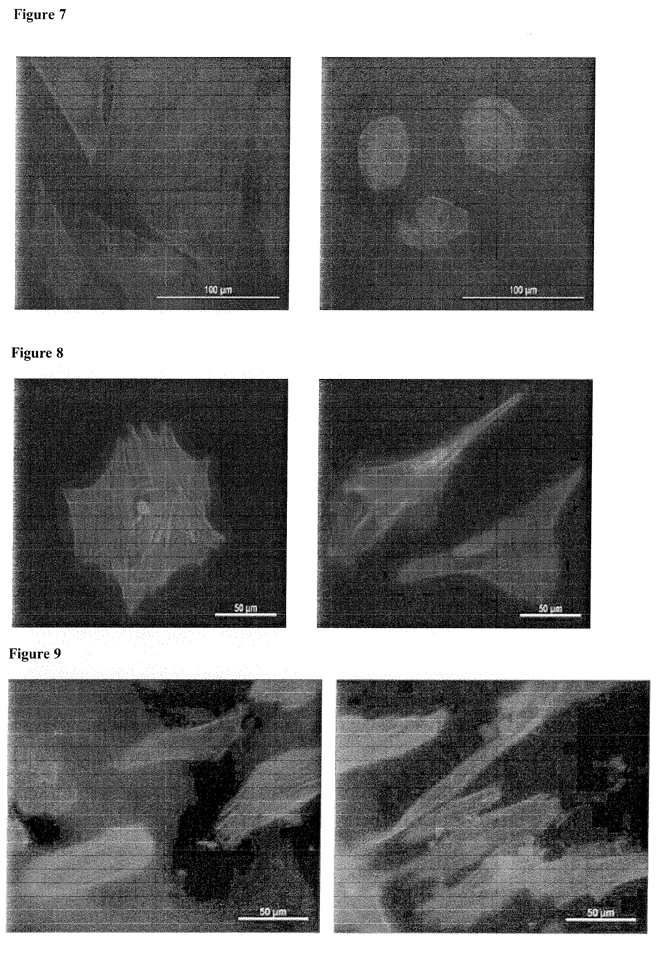

**Figure 7****:** Biological response of human osteosarcoma cells on scaffold option 2 (2200x1800x35). No alignment is observed.
**Figure 8****:** Biological response of human fibroblasts on non-grooved scaffolds. No alignment is observed.
**Figure 9****:** Biological response of human fibroblasts on scaffold option 1 **(2200x1800x300).** Alignment is observed due to the underlying topography.
**Figure 10****:** Biological response of human fibroblasts on scaffold option 2 (2200x1800x35). No alignment is observed.
**Figure 11****:** Biological response of human osteosarcoma on scaffolds option 1 and 2. Although no apparent directional growth was observed, the metabolic activity was significantly decreased for scaffold option 2 (2200x1800x35).

### Detailed Description of the Drawings

### Materials

Poly(glycolide-co-lactide) (PLGA) was purchased from PURAC Biomaterials, Netherlands. AlamarBlue® was purchased from BioSource International, Invitrogen, Dun Laoghaire, Ireland. Nano imprinted samples were fabricated by CRANN (Centre for Research on Adaptive Nanostructures and Nanodevices, Trinity College, Dublin). One of the nano imprint stamps (line width - 350nm, groove width - 250nm, groove depth - 35nm) had to be sourced externally from NIL Technology Denmark. µ-chamber 12 well culture slides for cell culture were purchased from Thistle Scientific Ltd, Glasgow.

Human osteosarcoma cells (SAOS2) were obtained from the European Collection of Cell Cultures, Salisbury, United Kingdom. Human Osteoblast cells were purchased from Lonza group Ltd, Switzerland. Bovine tenocytes were isolated from bovine tendons collected from a local abattoir. Human Tenocytes were kindly donation by Prof. Graham Riley (UEA, UK).

All other material was purchased from Sigma Aldrich unless otherwise stated.

### Methods

### Scaffold Fabrication Technique

### Electrospinning

The electrospinning set up consisted of a high voltage power supply (Gamma High Voltage, USA), a syringe pump (NE-1000, New Era Pump Systems Inc., USA) and a rotating drum. Poly(glycolide-co-lactide) (PLGA) was dissolved in chloroform to prepare an 8% w/v solution. The polymer solution was loaded into a syringe (Becton, Dickinson and Company, Ireland) fitted with an 18G stainless steel needle (Becton, Dickinson and Company, Ireland). The needle was subsequently connected to a high voltage power supply. Details of injection speed and voltage settings are reported in Table 1. The nanofibres were collected on metallic rotating drum with a diameter of 10cm, covered in tin foil. The drum was placed at a distance of 18cm away from the tip of the needle. For aligned samples a rotational speed was set to 1480rpm while randomly aligned samples were spun at 80rpm.

**Table 1 Electrospinning parameters used to produce aligned and random sheets of PLGA and PLA/PCL (Polylactic Acid/polycaprolactone)**

| **Material:** | **PLGA** |
|---|---|
| **Concentration:** | 8% w/v |
| **Solvent:** | Chloroform |
| **Voltage:** | 15 kV |
| **Distance:** | 18cm |
| **Injection Speed:** | 10µl/min |
| **Gauge Needle:** | 18G |

PLGA solution (8% w/v chloroform) was used to fabricate polymer films with no topographical features (i.e. smooth surface). As solvent casting method was employed to create thin polymer films. Briefly, the polymer solution was gently poured into the aluminium foil trays, avoiding bubble formation. The trays were covered with an aluminium foil cover to control the evaporation rate of the solvent.

### Nano Imprinting Lithography

Nano imprinting was carried out at CRANN (Centre for Research on Adaptive Nanostructures and Nanodevices, Trinity College, Dublin) facilities. The films were initially solvent cast by Proxy Biomedical and forwarded to CRANN for imprinting. The dimensions of the grooves are listed in Table 2 below.

**Table 2 List of nano-imprinted groove dimensions**

| | **Line Width (nm)** | **Groove Width (nm)** | **Groove Depth (nm)** | **Supplied By** |
|---|---|---|---|---|
| 1 | 1800 | 2200 | | CRANN |
| 2 | 1800 | 2200 | | CRANN |
| 3 | 1800 | 2200 | | CRANN |
| 4 | 250 | 350 | | CRANN |
| Control | N/A | N/A | N/A | Proxy Biomedical |

### Cell Culture

### Culture of Human osteosarcoma (SAOS2)

Human osteosarcoma (SAOS2) cells were routinely grown in McCoy's 5A basal media supplemented with 10% fetal bovine serum and 1% penicillin/streptomycin. Cells were subcultured when they reached 80% confluence (once a week) and maintained at 37°C and 5% carbon dioxide. Media was changed every 2-3 days. After 7 days the media was supplemented with ascorbic acid (50mg/L), dexamethasone (10 ng/L) and b-glycerophosphate (10mM) to differentiate the cells. The time point for this cell type was 5, 10, and 14 days.

### Culture of Human Osteoblasts

Natural human osteoblasts (Lonza, CC-2538) were cultured in Dulbecco's modified Eagle's medium (DMEM) supplemented with 10% Fetal Bovine Serum (FBS) and 1% penicillin/streptomycin in 75cm2 flasks. The osteoblast culture was maintained at 37°C in a humidified CO2 incubator until they were approximately 80% confluent, with media being changed every 3 days. At passages 4 and 5 cells were seeded (2 x 104 cells/cm2) on PLGA aligned and non-aligned electrospun scaffolds, solvent cast films and tissue culture cover slips. Cells were then assessed for with different assays at various time points. The time points for this cell type were 2, 7, 14, and 21 days.

### Culture of Bovine/Human Tenocytes

Bovine/human tenocytes cells were grown in Dulbecco's modified Eagle's medium (DMEM) supplemented 10% fetal bovine serum and 1% penicillin/streptomycin. Cells were subcultures when they reached 80% confluence (once a week) and maintained at 37°C and 5% carbon dioxide. Media was changed every 2-3 days. The time points for this cell type were 2, 5, and 7 days.

### Live/Dead® Staining

Live/Dead® Cell Viability Assay (BioSource International, Invitrogen, Dun Laoghaire, Ireland) was performed to examine the cell viability after seeding them on nanofibers. The protocol was adapted from the Live/Dead^{®} Viability/Cytotoxicity Kit *for mammalian cells* (Invitrogen). Briefly, the cells were washed with HBSS and the staining solution of Calcein and Ethidium Homodimer was added. The cells were incubated at 37°C for 30 to 45 minutes. Following staining, the cells were viewed using flurosence microscopy and analysed using ImageJ.

### AlamarBlue®

Cell metabolic activity was determined using alamarBlue® cell metabolic assay (BioSource International, Invitrogen, Dun Laoghaire, Ireland). To determine the cell metabolic activity throughout the study this assay was performed at predetermined time depending on cell type (mentioned above in cell culture section). Briefly, alamarBlue® dye was diluted with Hank's Balance Salt Solution, to make a 10% (v/v) alamarBlue® solution. Media was removed from each well and 0.5ml Alamar Blue® solution was added to each well. After an incubation time of 1.5 hours at 37°C, the absorbance was measured at wavelengths of 550 and 595nm using a micro plate reader (Varioskan Flash, Thermo Scientific). The level of metabolic activity was calculated using the "simplified method of calculating per cent reduction" according to the supplier's protocol.

### Immunocytochemistry

Quantification of cell alignment, density, and morphology was carried out using immunocytochemistry. Cell nuclei stained with DAPI and f-actin filaments stained with rhodamine phalloidin. Briefly, cells were fixed by incubation with 4% paraformaldehyde for 5mins at room temperature. The cells were then permeabilised by washing with 0.2% Triton X. The cells were then incubated with DAPI for 5mins, and after with rhodamine phalloidin for one hour. Cell alignment was evaluated using the 'count tool' in ImagePro. The 'count tool' measured the area, aspect, and alignment of the DAPI stained nuclei.

### Migration Assay

Collagen gel was made by mixing collagen solution 1mg/ml and 2M NaOH, adjusting concentration to ensure pH 7.0. A collagen gel/culture media/cell solution was prepared by mixing the collagen gel and media/cells suspension at a ratio of 1:1. The collagen/cells solution is then placed at the edge of the scaffold. At each time point cells were stained with DAPI and the distance between the nuclei and the edge of the scaffold measured.

### Taqman Analysis

Taqman microarray was used to evaluate the regulation of gene expression. A selection of genes associated with phenotype, adhesion, migration, apoptosis and extracellular matrix protein, listed in Table 3, was investigated. RNA extraction was carried out at the NFB using standard RNA extraction protocols. The Taqman analysis was carried out by Prof. Graham Riley at the University of East Anglia.

**Table 3 List of genes investigated using Taqman Microarray.**

| **Phenotype** | **Adhesion** | **Migration** | **Apoptosis** | **ECM** |
|---|---|---|---|---|
| Tenomodulin | Integrin a1 | RAC1 | Caspase 3 | Collagen I |
| Scleraxis | Integrin a2 | RhoA | Caspase 6 | Collagen III |
| Tenascin C | Integrin a3 | ROCK | Caspase 7 | Collagen IV |
| Decorin | Integrin a4 | Calpain 1 | Caspase 8 | Collagen V |
| TGFβ2 | Integrin a5 | Calpain 2 | Caspase 9 | Collagen VI |
| Follistatin | Integrin b1 | | | Collagen XII |
| osteopontin | Vinculin | | | |
| bone sialoprotein | Paxillin | | | |
| alkaline phosphatase | FAK | | | |
| osteocalcin | | | | |
| cartilage oligomeric protein | | | | |
| lumican | | | | |
| versican | | | | |
| RUNX2 | | | | |
| TGF-β1 | | | | |

### Experimental Approach:

**Cells:** Human fibroblasts and human and bovine tenocytes were chosen as representative cells for soft tissue repair. Human osteosarcoma and human osteoblasts were chosen as representative cells for hard tissue repair.

**Preliminary Results:** Solvent casting was used to fabricate smooth films. Electrospinning was used to fabricate non-aligned and aligned electro-spun mats. A thermal process was used to create porosity insolvent-cast and electro-spun scaffolds.

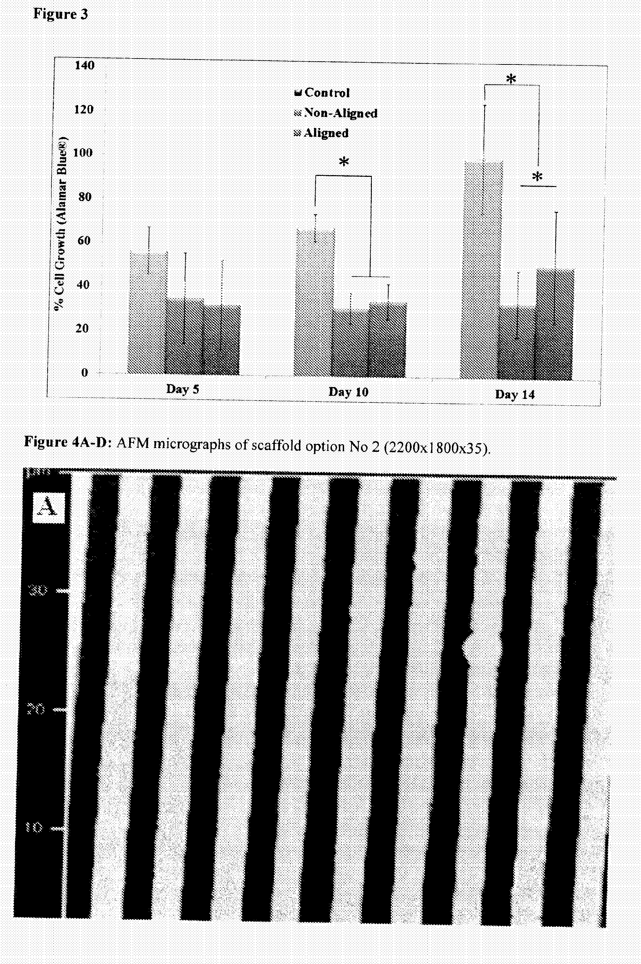

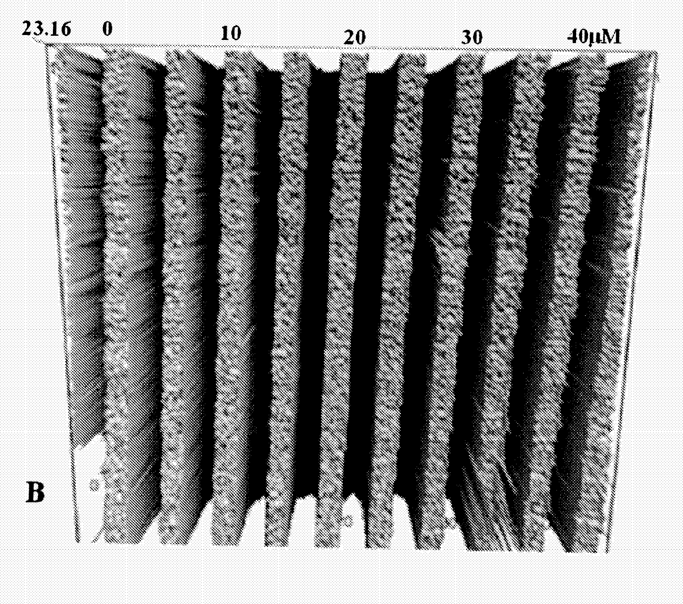

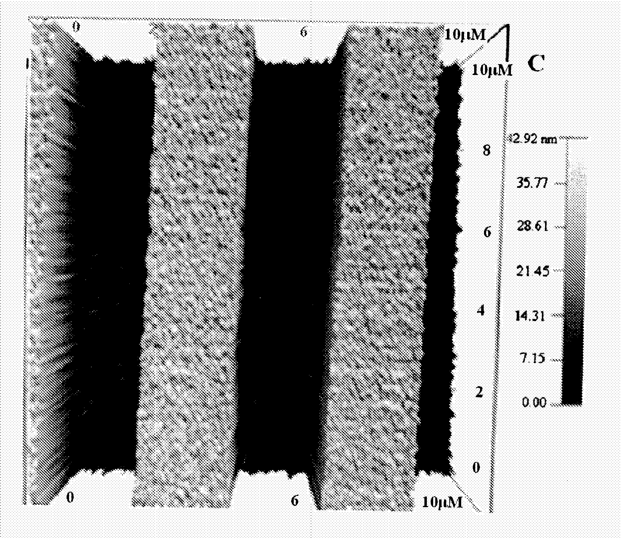

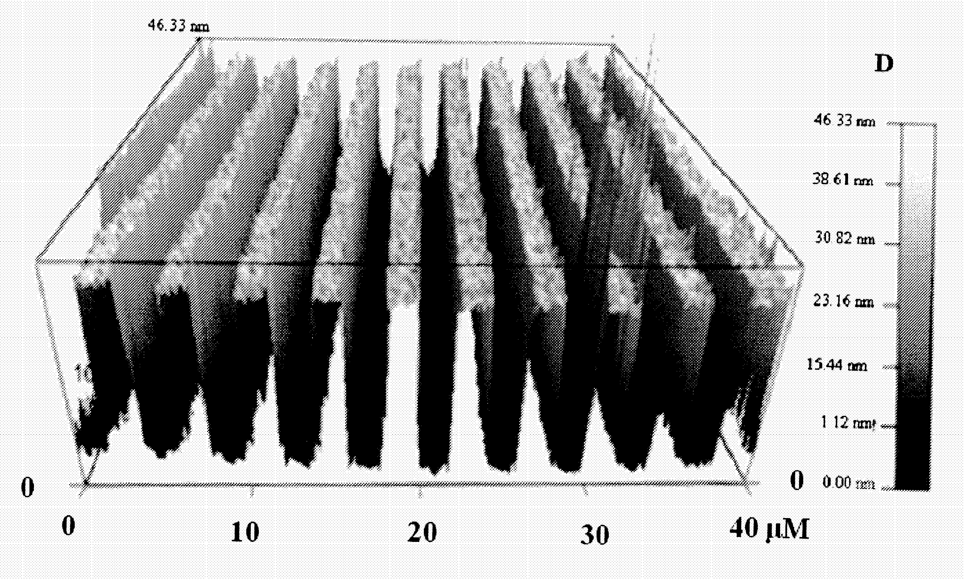

The reduction in metabolic activity and the poor control over scaffold architecture lead us to fabricate nano-grooved samples. Table 1 summarises the dimensions of the grooved samples, whilst Figure 4A - D shows an example of a non-grooved scaffold.

**Table 1: Dimensions of the nano-grooved solvent casted films.**

| Options | Groove width (nm) | Line width (nm) | (Groove depth (nm) |
|---|---|---|---|
| **1** | 2200 | 1800 | 300 |
| **2** | 2200 | 1800 | 35 |
| **3** | 2000 | 2000 | 2000 |
| **4** | 300 | 300 | 300 |
| **5** | 35 | 35 | 50 |

**Taqman gene assay:** 3 housekeeping and other 45 genes are investigated for tenocytes and osteoblasts grouped as follows: phenotype; extracellular matrix molecules; apoptosis; migration; attachment; proliferation; immune response; pain.

**Live/dead assay or FACS** are used to correlate the metabolic activity to number of live and dead cells.

**Migration assays:** are used to evaluate whether there is an ideal topography that will promote cellular migration

**Small animal study:** are necessary to evaluate functional neotissue formation. **Multiplex ELISA:** is used to evaluate protein expression

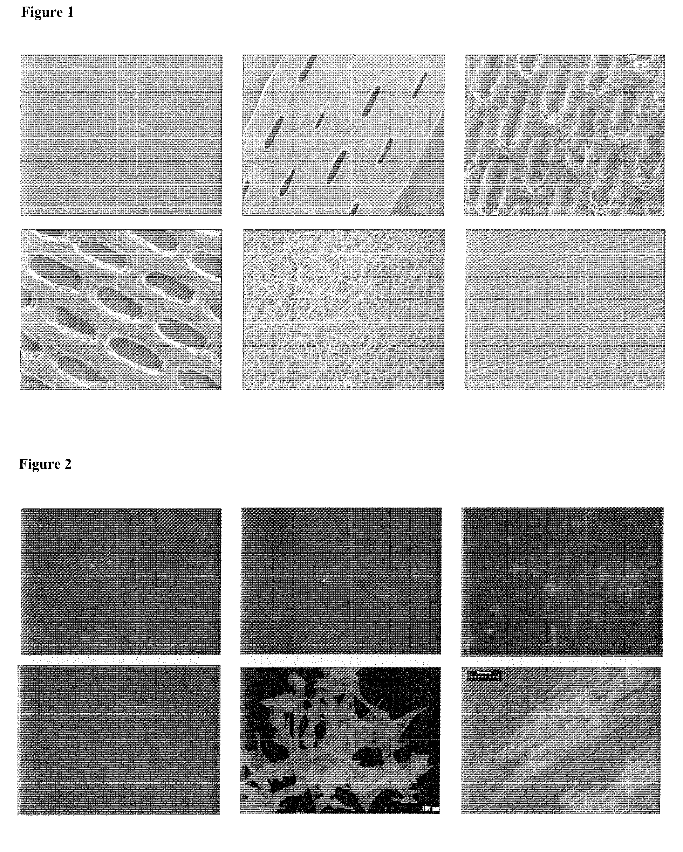

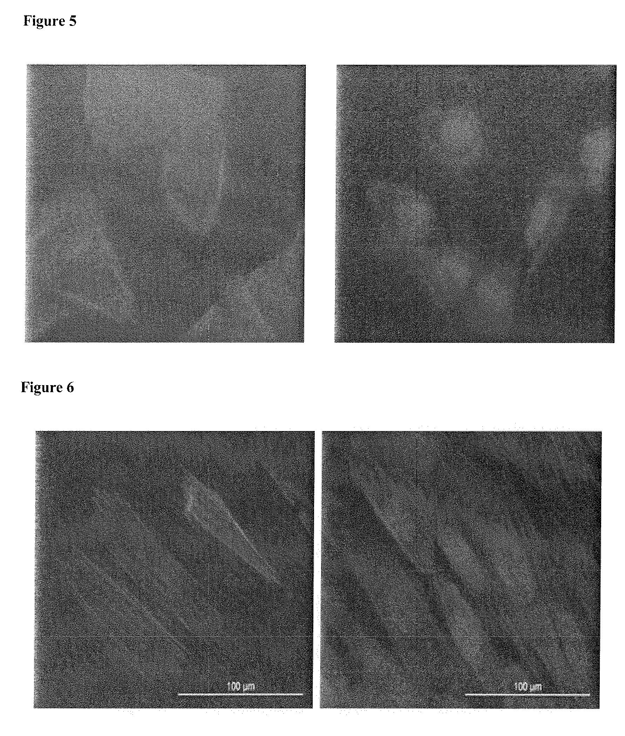

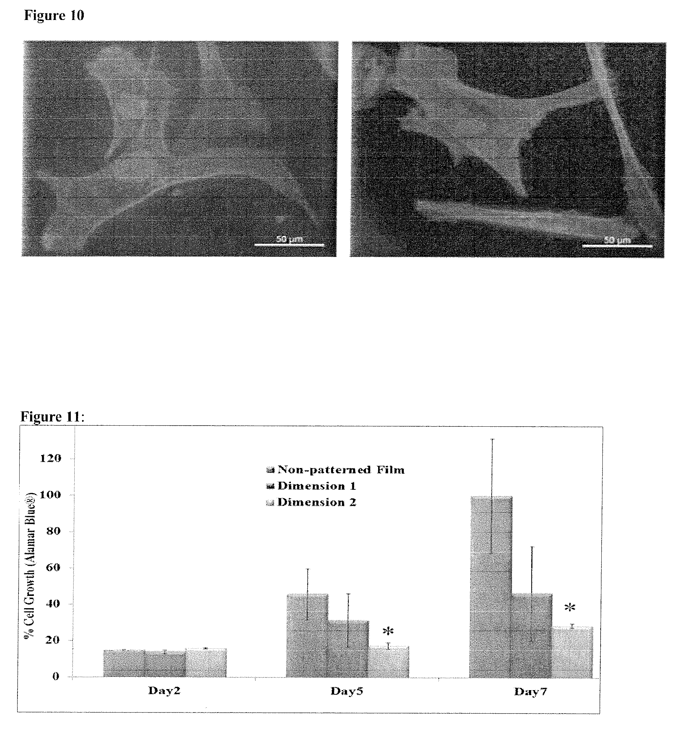

As can be seen from Figure 2 human osteosarcoma cells attach only on non-aligned or aligned electro-spun scaffolds but it is only an aligned electro-spun scaffold that promotes cell alignment in the direction of the substrate topography. This a critical requirement for growing tissue because non-aligned cells results in tissue which is not functional. Figure 3 shows that cells exposed to nano-textured scaffolds exhibit significantly reduced metabolic activity and prolonged culture. Figure 6 shows that human osteosarcoma cells grown on a grooved scaffold of the invention align in the direction of the underlying topography. It is significant that on a grooved scaffold where the groove depth is shallower (35nm as compared to 300nm) no such alignment can be seen. Similarly, human fibroblasts also align in the direction of the underlying topography with a groove depth of 300nm but not with a grooved depth of 35nm. Figure 11 shows that whilst metabolic activity is highest on a non-patterned film, the deeper grooved film (dimension 1) shows higher metabolic activity than the lower grooved scaffold. Similarly, Options 3, 4 and 5 from Table 1 do not result in aligned cells with the requisite level of cell metabolism. Thus overall a groove depth of approximately 300nm leads to both aligned cells and acceptable metabolic activity.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

### References

28. Desai, T.A., Micro- and nanoscale structures for tissue engineering constructs. Med. Eng. Phy., 2000. 22(9): p. 595-606.
29. Cornwell, K.G., B. Downing, and G.D. Pins, Characterizing fibroblast migration on discrete collagen threads for applications in tissue regeneration. Journal of Biomedical Materials Research Part A, 2004. 71A(1): p. 55-62.
30. Zeltinger, J., et al., Effect of Pore Size and Void Fraction on Cellular Adhesion, Proliferation, and Matrix Deposition. Tissue Engineering, 2001. 7(5): p. 557-572.
31. Carnell, L.S., et al., Aligned Mats from Electrospun Single Fibers. Macromolecules, 2008.
32. Zhong, S., et al., An aligned nanofibrous collagen scaffold by electrospinning and its effects on in vitro fibroblast culture. Journal of Biomedical Materials Research Part A, 2006. 79A(3): p. 456-463.
33. Schnell, E., et al., Guidance of glial cell migration and axonal growth on electrospun nanofibers of poly-[epsilon]-caprolactone and a collagen/poly-[epsilon]-caprolactone blend. Biomaterials, 2007. 28(19): p. 3012-3025.
34. Stitzel, J., et al., Controlled fabrication of a biological vascular substitute. Biomaterials, 2006. 27(7): p. 1088-1094.
35. Lee, L.J., Polymer Nanoengineering for Biomedical Applications. Annals of Biomedical Engineering, 2006. 34(1): p. 75-88.
36. Hollister, S.J., Porous scaffold design for tissue engineering. nature materials, 2005. 4: p. 518-524.
37. Hutmacher, D.W., Scaffold design and fabrication technologies for engineering tissues-state of the art and future prespectives. Journal of Biomaterials Science. Polymer Edition, 2001. 12(1): p. 107-124.
38. Stevens, M. and J. George, Exploring and engineering the cell surface interface. Science, 2005. 310: p. 1135-1138.
39. Xia, Y., Nanomaterials at work in biomedical research. Nature Materials, 2008. 7: p. 758-760.
40. Barnes, C.P., et al., Nanofiber technology: Designing the next generation of tissue engineering scaffolds. Advanced Drug Delivery Reviews, 2007. 59(14): p. 1413-1433.
41. Chew, S., et al., The Role of Electrospinning in the Emerging Field of Nanomedicine. Curr Pharm Des, 2006. 12(36): p. 4751-4770.
42. Park, H., et al., Nanofabrication and microfabrication of functional materials for tissue engineering. Tissue Engineering, 2007. 13(8): p. 1867-1877.
43. Yao, L., et al., Effect of functionalized micropatterned PLGA on guided neurite growth. Acta Biomaterialia, 2009. 5(2): p. 580-588.
44. Lamers, E., et al., The influence of nanoscale grooved substrates on osteoblast behavior and extracellular matrix deposition. Biomaterials, 2010. 31(12): p. 3307-3316.
45. Zhu, J., et al., The regulation of phenotype of cultured tenocytes by microgrooved surface structure. Biomaterials, 2010. 31(27): p. 6952-6958.
46. Brunetti, V., et al., Neurons sense nanoscale roughness with nanometer sensitivity. Proceedings of the National Academy of Sciences, 2010. 107(14): p. 6264-6269.

## Claims

1. A scaffold for the growth of tissue comprising a film of supporting material, wherein the film has a plurality of nano-grooves on its surface.

2. A scaffold as claimed in claim 1 wherein the groove width of about 2,200nm.

3. A scaffold as claimed in either claim 1 or claim 2 wherein the grooves have a line width of about 1,800nm.

4. A scaffold as claimed in claim 1, 2 or 3 wherein the groove depth is about 300nm.

5. A scaffold as claimed in any preceding claim wherein the supporting material is (poly)glycolide-co-lactide or a PLA/PCL.

6. A scaffold as claimed in any preceding claim for the growth of human osteosarcoma cells wherein the grooves have a width of 2,200nm, a line width of 1,800nm and a groove depth of 300nm.

7. A scaffold as claimed in any of claims 1 to 5 for the growth of human fibroblasts wherein the grooves have a width of 2,200nm, a line width of 1,800nm and a groove depth of 300nm.

8. A scaffold as claimed in any of claims 1 to 5 for the growth of tenocytes wherein the grooves have a width of 2,200nm, a line width of 1,800nm and a groove depth of 300nm.

9. Use of a tissue scaffold having nano-grooves on the surface thereof in a method of repairing or replacing tissue in a patient.

10. A method of repairing or replacing tissue in a patient comprising use of a tissue scaffold having nano-grooves on the surface thereof.

11. A method as claimed in claim 10, wherein the human osteosarcoma cells and the nano-grooves on the mat have a width of 2,200nm, a line width of 1,800nm and a groove depth of 300nm.

12. A method as claimed in claim 10 wherein the cells are human fibrblasts and the grooves have a groove width of 2,200nm, a line width of 1,800nm and a groove depth of 300nm.

13. A method as claimed in any of claims 10 to 12 wherein the scaffold is a (poly)glycolide-co-lactide or a PLA/PCL scaffold.
